# EUROPEAN PATENT APPLICATION

(11) **EP 4 425 165 A1**
(43) Date of publication of application: **04.09.2024**
(21) Application number: 22886781.8
(22) Date of filing: 18.10.2022
(51) Int. Cl.: G01N 27/416, C12M 1/34

(54) **ELECTROCHEMICAL REACTION DEVICE AND METHOD FOR ELECTROCHEMICALLY ANALYZING GLUCOSE**

(30) Priority: 28.10.2021 JP 2021176184
(71) Applicant: Panasonic Intellectual Property Management Co., Ltd., Osaka-shi, Osaka 540-6207 (JP)
(72) Inventor: TSUJI, Kiyotaka, Kadoma-shi Osaka 571-0057 (JP)
(74) Representative: Grünecker Patent- und Rechtsanwälte PartG mbB
(86) International application number: PCT/JP2022/038666
(87) International publication number: WO 2023/074453

(57) **Abstract**

The present disclosure provides an electrochemical reaction device that is advantageous from the viewpoint of energy efficiency while continuously oxidizing NAD and decomposing glucose. An electrochemical reaction device (1a) of the present disclosure includes a first tank (10), a second tank (20), a membrane (16), and a voltage application device (30). The first tank (10) accommodates glucose dehydrogenase and nicotinamide adenine dinucleotide. Further, a working electrode (11) is disposed in the first tank (10). A counter electrode (21) is disposed in the second tank (20). The voltage application device (30) applies a voltage between the working electrode (11) and the counter electrode (21). The membrane (16) separates the inside of the first tank (10) and the inside of the second tank (20) from each other. The membrane (16) blocks permeation of the glucose dehydrogenase and the nicotinamide adenine dinucleotide and has ion conductivity.

## Description

### Technical Field

The present disclosure relates to an electrochemical reaction device and an electrochemical decomposition method for glucose.

### Background Art

In the related art, it has been known that glucose can be decomposed by using glucose dehydrogenase and nicotinamide adenine dinucleotide.

For example, PTL 1 describes a glucose biosensor including a working electrode formed such that glucose dehydrogenase and nicotinamide adenine dinucleotide are adsorbed and immobilized on a predetermined electrode surface.

PTL 2 describes an enzyme electrode including a conductive substrate and an enzyme. The enzyme is formed of associated proteins of a first enzyme such as glucose dehydrogenase and a second enzyme such as diaphorase. When glucose is allowed to act on this enzyme electrode, the glucose is oxidized by a catalytic action of glucose dehydrogenase in the coexistence of nicotinamide adenine dinucleotide (NAD⁺). As a result, gluconolactone and reduced nicotinamide adenine nucleotide (NADH) are generated. NADH is readily oxidized by the catalytic action of diaphorase that is present in the physical vicinity of glucose dehydrogenase in the presence of an oxidized electron transfer mediator. As a result, nicotinamide adenine dinucleotide and a reduced electron transfer mediator are generated.

### Citation List

### Patent Literature

PTL 1: Japanese Unexamined Patent Application Publication No. 4-370755
PTL 2: Japanese Unexamined Patent Application Publication No. 2007-163268

### Summary of Invention

### Technical Problem

The present disclosure provides an electrochemical reaction device that is advantageous from the viewpoint of energy efficiency while continuously oxidizing nicotinamide adenine dinucleotide and decomposing glucose.

### Solution to Problem

According to the present disclosure, there is provided an electrochemical reaction device including a first tank in which glucose dehydrogenase and nicotinamide adenine dinucleotide are accommodated and a working electrode is disposed, a second tank in which a counter electrode is disposed, a membrane which separates an inside of the first tank and an inside of the second tank from each other, blocks permeation of the glucose dehydrogenase and the nicotinamide adenine dinucleotide, and has ion conductivity, and a voltage application device which applies a voltage between the working electrode and the counter electrode.

### Advantageous Effects of Invention

The electrochemical reaction device of the present disclosure is advantageous from the viewpoint of energy efficiency while continuously oxidizing nicotinamide adenine dinucleotide and decomposing glucose.

### Brief Description of Drawings

[Fig. 1] Fig. 1 is a view showing an example of an electrochemical reaction device according to an embodiment.
[Fig. 2] Fig. 2 is a view showing an electrochemical reaction device according to a comparative example.
[Fig. 3] Fig. 3 is a graph showing a relationship between the voltage application time and the current when a device of Example 1 is used.
[Fig. 4] Fig. 4 is a graph showing a relationship between the voltage application time and the current when a device of Example 2 is used.
[Fig. 5] Fig. 5 is a graph showing a relationship between the voltage application time and the current when a device of Comparative Example 1 is used.
[Fig. 6] Fig. 6 is a graph showing a relationship between the voltage application time and the current when a device of Comparative Example 2 is used.
[Fig. 7] Fig. 7 is a graph showing a relationship between the voltage application time and the current when a device of Comparative Example 3 is used.

### Description of Embodiments

### (Underlying knowledge forming basis of the present disclosure)

Glucose dehydrogenase (GDH) functions as a catalyst in a D-glucose decomposition reaction represented by Formula (1) in which nicotinamide adenine dinucleotide (NAD) functions as a coenzyme.

D-glucose + NAD⁺ → D-glucono-1,5-lactone + NADH + H⁺ Formula (1)

When the concentration of glucose in a predetermined sample is measured using the above-described reaction, for example, the concentration of NADH generated per unit time is measured. In this case, the amount of NAD⁺ consumed for measuring the concentration of glucose is not significantly large. Meanwhile, when glucose in a liquid containing glucose is decomposed by using the above-described reaction, NAD may be difficult to add at a concentration commensurate with the amount of glucose contained in the liquid. This is because the concentration of NAD dissolved in a liquid has its upper limit.

Therefore, when NADH generated in the above-described reaction is oxidized to generate NAD⁺, glucose contained in a liquid at a high concentration is considered to be decomposed by adding a small amount of NAD.

Meanwhile, in the examination conducted by the present inventor, it is advantageous that the energy efficiency is high when glucose is decomposed while NADH generated in the above-described reaction is continuously oxidized by an electrochemical method. Here, as a result of intensive examination repeatedly conducted by the present inventor, a configuration of an electrochemical reaction device that is advantageous from the viewpoint of the energy efficiency while continuously oxidizing nicotinamide adenine dinucleotide and decomposing glucose has been newly found. The present inventor has completed the electrochemical reaction device of the present disclosure based on this new knowledge.

### (Embodiments of present disclosure)

Hereinafter, embodiments of the present disclosure will be described with reference to the accompanying drawings. Further, all the embodiments described below show comprehensive or specific examples. The numerical values, the shapes, the materials, the constituent elements, the positions where the constituent elements are disposed, the connection forms, the process conditions, the steps, the order of the steps, and the like described in the following embodiments are merely examples and do not limit the present disclosure. Further, the constituent elements that are not described in the independent claims showing the most significant concept, among the constituent elements in the following embodiments, are described as optional constituent elements. Further, each view is a schematic view and is not necessarily strictly shown.

### (Embodiments)

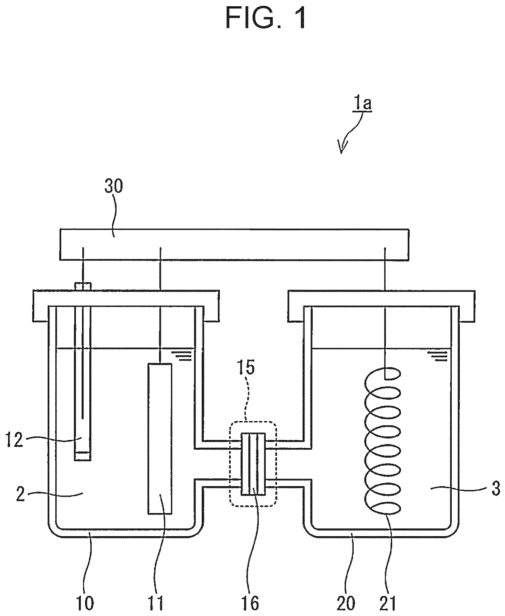

Fig 1 is a view schematically showing an electrochemical reaction device 1a according to an embodiment. The electrochemical reaction device 1a includes a first tank 10, a second tank 20, a membrane 16, and a voltage application device 30. The first tank 10 accommodates glucose dehydrogenase and nicotinamide adenine dinucleotide. Further, a working electrode 11 is disposed in the first tank 10. A counter electrode 21 is disposed in the second tank 20. The voltage application device 30 is a device for applying a voltage between the working electrode 11 and the counter electrode 21. The membrane 16 separates the inside of the first tank 10 and the inside of the second tank 20 from each other. Further, the membrane 16 blocks permeation of glucose dehydrogenase and nicotinamide adenine dinucleotide and has ion conductivity.

As shown in Fig. 1, for example, a first liquid 2 is accommodated in the first tank 10. The first liquid 2 contains glucose dehydrogenase and nicotinamide adenine dinucleotide. At least a part of the working electrode 11 is in contact with the first liquid 2.

The first liquid 2 may contain, for example, glucose. Alternatively, a raw material containing glucose may be added to the first liquid 2.

As shown in Fig. 1, for example, a second liquid 3 is accommodated in the second tank 20. At least a part of the counter electrode 21 is in contact with the second liquid 3.

An electrochemical decomposition method for glucose, including, for example, the following step (I) carried out using the electrochemical reaction device 1a can be provided. In the step (I), for example, a voltage is applied between the working electrode 11 and the counter electrode 21 by the voltage application device 30.
(I) A voltage is applied between the working electrode 11 disposed in the first tank 10 in which a liquid containing glucose dehydrogenase, nicotinamide adenine dinucleotide, and glucose is accommodated, and the counter electrode 21.

Glucose dehydrogenase functions as a catalyst and nicotinamide adenine dinucleotide functions as a coenzyme. As a result, the reaction represented by Formula (1) occurs. In this manner, glucose in the first tank 10 is decomposed. Meanwhile, when a voltage is applied between the working electrode 11 and the counter electrode 21 by the voltage application device 30, reduced nicotinamide adenine dinucleotide (NADH) generated in the reaction represented by Formula (1) is oxidized by the working electrode 11. In this manner, oxidized nicotinamide adenine dinucleotide (NAD⁺) is generated. Since NAD⁺ is generated due to continuous oxidation of NADH, glucose can be continuously decomposed even when the first liquid 2 does not contain a large amount of nicotinamide adenine dinucleotide.

The membrane 16 blocks permeation of glucose dehydrogenase and nicotinamide adenine dinucleotide. Therefore, NAD⁺ that is present inside the first tank 10 remains inside the first tank 10 and thus is unlikely to be guided to the inside of the second tank 20. Accordingly, generation of NADH by NAD⁺ being reduced by the counter electrode 21 in the electrochemical reaction device 1a is likely to be prevented. Therefore, the electrochemical reaction device 1a is advantageous from the viewpoint of energy efficiency while continuously oxidizing nicotinamide adenine dinucleotide and decomposing glucose. Since the membrane 16 has ion conductivity, for example, predetermined ions permeate through the membrane 16 so that the electrical neutrality of the first liquid 2 and the second liquid 3 is maintained.

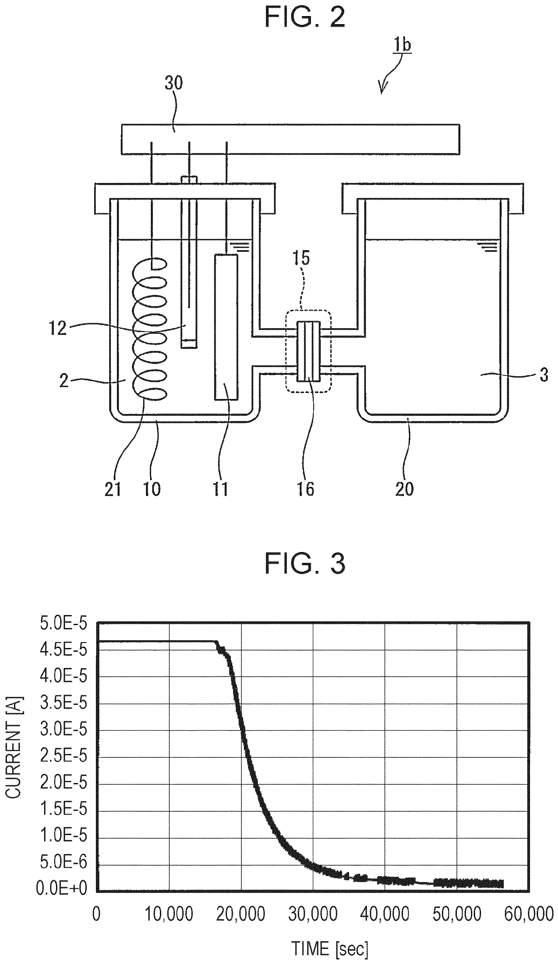

Fig. 2 is a view schematically showing an electrochemical reaction device 1b according to a comparative example. An electrochemical reaction device 1b has the same configuration as that of the electrochemical reaction device 1a except that the counter electrode 21 is disposed in the first tank 10 in place of the second tank 20. Since in the electrochemical reaction device 1b, the counter electrode 21 is disposed in the first tank 10, NADH is likely to be generated by NAD⁺ being reduced by the counter electrode 21. This is disadvantageous from the viewpoint of the energy efficiency.

The material forming the working electrode 11 is not limited to a specific material. The working electrode 11 has, for example, a surface formed of platinum. According to such a configuration, NADH is likely to be directly oxidized by the working electrode 11, and NAD⁺ required for decomposition of glucose in the reaction represented by Formula (1) is likely to be supplied. Therefore, glucose can be efficiently decomposed. The material forming the surface of the working electrode 11 may be gold (Au), a carbon material such as glassy carbon, graphite, or boron-doped diamond, or tin-added indium oxide (ITO).

The shape of the working electrode 11 is not limited to a specific shape. The working electrode 11 may be linear, plate-shaped, or mesh-shaped or may be a fiber assembly. From the viewpoint of efficiency of glucose decomposition, it is advantageous that the surface area of the working electrode 11 is large.

The material forming the counter electrode 21 is not limited to a specific material. The counter electrode 21 has, for example, a surface formed of platinum. According to such a configuration, electrons are likely to be released from the counter electrode 21 to maintain the electrical neutrality of the first liquid 2 and the second liquid 3 in response to the oxidation of NADH, and glucose decomposition can be efficiently carried out. The material forming the surface of the counter electrode 21 may be gold (Au), a carbon material such as glassy carbon, graphite, or boron-doped diamond, or tin-added indium oxide (ITO).

The shape of the counter electrode 21 is not limited to a specific shape. The counter electrode 21 may be linear, plate-shaped, or mesh-shaped or may be a fiber assembly. From the viewpoint of efficiency of glucose decomposition, it is advantageous that the surface area of the counter electrode 21 is large.

The material forming the membrane 16 is not limited to a specific material as long as the material blocks permeation of GDH and NAD and has ion conductivity. The membrane 16 contains, for example, a polymer having a perfluoro side chain including a sulfonic acid group. In such a configuration, predetermined ions are likely to quickly move through the membrane 16, and thus glucose decomposition can be efficiently carried out. Nafion (registered trademark) is known as such a polymer. The material forming the membrane 16 may be porous glass or porous silicon.

The electrochemical reaction device 1a includes, for example, a connection portion 15. The connection portion 15 is configured such that the membrane 16 is disposed between a tubularly protruding portion on a side portion of the first tank 10 and a tubularly protruding portion on a side portion of the second tank 20.

The first tank 10 and the second tank 20 accommodate, for example, a phosphate buffer solution. The phosphate buffer solution is, for example, contained in the first liquid 2 and the second liquid 3. In such a configuration, a rapid change in pH of the second liquid 2 and the third liquid 3 can be prevented, and thus glucose decomposition is likely to be stably carried out.

The voltage application device 30 is not limited to a specific device as long as the device can apply a voltage between the working electrode 11 and the counter electrode 21. The voltage application device 30 is, for example, a potentiostat. The electrochemical reaction device 1a includes, for example, a reference electrode 12. The reference electrode 12 is disposed in the first tank 10, and at least a part of the reference electrode 12 is in contact with the first liquid 2. The working electrode 11, the reference electrode 12, and the counter electrode 21 are respectively electrically connected to a working electrode terminal, a reference electrode terminal, and a counter electrode terminal of the potentiostat. The reference electrode 12 may be omitted.

The raw material containing glucose to be treated by the electrochemical reaction device 1a is not limited to a specific raw material. The raw material is, for example, a food raw material, and more specific examples thereof include egg white. In the electrochemical reaction device 1a, glucose contained in egg white can be decomposed, and thus a desired product is likely to be obtained. The reason for this is considered to be that since permeation of components contained in egg white is likely to be blocked by the membrane 16, reactions involving the components are unlikely to occur.

### EXAMPLES

The electrochemical reaction device of the present disclosure will be described in more detail based on examples. Further, the electrochemical reaction device of the present disclosure is not limited to the following examples.

### <Example 1>

A device of Example 1 corresponding to an electrochemical reaction device 1a shown in Fig. 1 was prepared. In this device, an electrode in which a platinum wire having a wire diameter of 0.5 mm and a length of 23 cm was wound into a coil was used as a working electrode. A silver-silver chloride (Ag/AgCl) electrode was used as a reference electrode. An electrode in which a platinum wire having a wire diameter of 0.5 mm and a length of 23 cm was wound into a coil was used as a counter electrode. A three-electrode cell was configured by electrically connecting the working electrode, the reference electrode, and the counter electrode respectively to a working electrode terminal, a reference electrode terminal, and a counter electrode terminal of a potentiostat, using OctoStat30 provided by Ivium Technologies B.V. as the potentiostat. An ion exchange membrane Nafion 117 provided by MTI Corporation was used as the membrane separating the inside of the first tank and the inside of the second tank from each other in the device of Example 1.

Phosphate-Buffer Saline (PBS) which is a phosphate buffer solution having a pH of 7.4 provided by Nippon Gene Co., Ltd. was accommodated in the second tank in which the counter electrode was disposed. A part of the counter electrode was in contact with PBS in the second tank. The liquid amount of PBS accommodated in the second tank was 20 milliliters (mL).

A treatment liquid of Example 1 was prepared by dissolving glucose, reduced nicotinamide adenine dinucleotide, and glucose dehydrogenase in the above-described phosphate buffer solution separately prepared. D(+)-glucose provided by FUJIFILM Wako Pure Chemical Corporation was used as glucose. β-Nicotinamide Adenine Dinucleotide Disodium Salt (reduced form) provided by Nacalai Tesque Inc. was used as the reduced nicotinamide adenine dinucleotide (NAD). Glucose Dehydrogenase (GDH) provided by FUJIFILM Wako Pure Chemical Corporation was used as glucose dehydrogenase. The concentration of glucose in the treatment liquid of Example 1 was 1×10⁻³ [mol/liter (L)]. The concentration of NAD in the treatment liquid of Example 1 was 1×10⁻³ [mol/liter (L)]. The concentration of GDH in the treatment liquid of Example 1 was 0.2 [unit/mL]. 20 mL of the treatment liquid of Example 1 was accommodated in the first tank. A part of the working electrode and a part of the reference electrode were in contact with the treatment liquid of Example 1 in the first tank.

A constant voltage was continuously applied to the working electrode such that a difference obtained by subtracting the potential of the reference electrode from the potential of the working electrode in the device of Example 1 reached +1.0 V. In this case, the current flowing through the counter electrode was measured. The application of the voltage to the working electrode and the measurement of the current flowing through the counter electrode were performed at room temperature (15°C to 27°C). In addition, the treatment liquid was stirred with a stirrer.

The treatment liquid was sampled at a predetermined timing during a period in which a constant voltage was continuously applied to the working electrode. Fifty microliters (50 µL) of the sampled treatment liquid was added dropwise to a urine sugar inspection part of a urine test strip new Uriace BT (manufactured by Terumo Corporation), a change in color of the urine sugar inspection part was visually confirmed, and the presence or absence and the degree of glucose decomposition were confirmed. As a result, it was confirmed that in a case where the concentration of glucose was 0.1×10⁻³ [mol/liter (L)] or less, almost no discoloration was found in the urine sugar inspection part of the present urine test strip even when the treatment liquid was added dropwise to the part. In addition, it was confirmed that the urine sugar inspection part was discolored to deep blue when the concentration of glucose was greater than 0.1×10⁻³ [mol/liter (L)], and thus both cases were visually distinguishable from each other.

Fig. 3 is a graph showing the relationship between the time during which the voltage was applied to the working electrode and the measured value of the current flowing through the counter electrode in the device of Example 1. In Fig. 3, the vertical axis represents the measured value of the current flowing through the counter electrode, and the horizontal axis represents the time during which the voltage was applied to the working electrode. As shown in Fig. 3, the value of the current flowing through the counter electrode during a predetermined period from the start of the voltage application was about 470 µA, which was substantially constant. However, the current flowing through the counter electrode after about 4.5 hours from the start of the voltage application was gradually decreased, and the current flowing through the counter electrode after about 15 hours from the start of the voltage application was decreased to about 1 µA. The treatment liquid after about 15 hours from the start of the voltage application did not change the color of the urine sugar inspection part of the urine test strip, and this result confirmed that the glucose was decomposed to a concentration less than or equal to the detection limit.

### <Example 2>

A device of Example 2 was prepared in the same manner as in Example 1.

Phosphate-Buffer Saline (PBS) which is a phosphate buffer solution having a pH of 7.4 provided by Nippon Gene Co., Ltd. was accommodated in the second tank in which the counter electrode was disposed. A part of the counter electrode was in contact with PBS in the second tank. The liquid amount of PBS accommodated in the second tank was 8.1 mL.

Egg yolks and egg whites of commercially available eggs were separated from each other manually. The separated egg whites were stirred manually using two chopsticks to cut off a highly viscous part to the extent that bubbles were not made. Thereafter, egg whites were filtered through gauze to remove a solid part, thereby obtaining an egg white liquid. Typical egg white contains about 0.7% of glucose on a mass basis, and the concentration of the glucose in the egg white is about 22×10⁻³ [mol/L].

NAD was dissolved in the above-described phosphate buffer solution separately prepared to prepare a NAD solution with a concentration of 40×10⁻³ [mol/L]. Further, GDH was dissolved in the above-described phosphate buffer solution separately prepared to prepare a GDH solution with a concentration of 80 [unit/mL]. 8.1 mL of a treatment liquid of Example 2 which was obtained by mixing 7.2 mL of the egg white liquid, 0.8 mL of the NAD solution, and 0.1 mL of the GDH solution was accommodated in the first tank of the device of Example 2. The concentration of glucose of the treatment liquid of Example 2 was about 20×10⁻³ [mol/L]. The concentration of NAD of the treatment liquid of Example 2 was about 4×10⁻³ [mol/L]. The content of GDH in the treatment liquid of Example 2 was about 8 units.

A constant voltage was continuously applied to the working electrode such that a difference obtained by subtracting the potential of the reference electrode from the potential of the working electrode in the device of Example 2 reached +1.0 V. In this case, the current flowing through the counter electrode was measured. The application of the voltage to the working electrode and the measurement of the current flowing through the counter electrode were performed at room temperature (15°C to 27°C). In addition, the treatment liquid was stirred with a stirrer to the extent that the treatment liquid was not bubbled. The treatment liquid was sampled at a predetermined timing, and the presence or absence and the degree of glucose decomposition were confirmed using a urine test strip in the same manner as in Example 1.

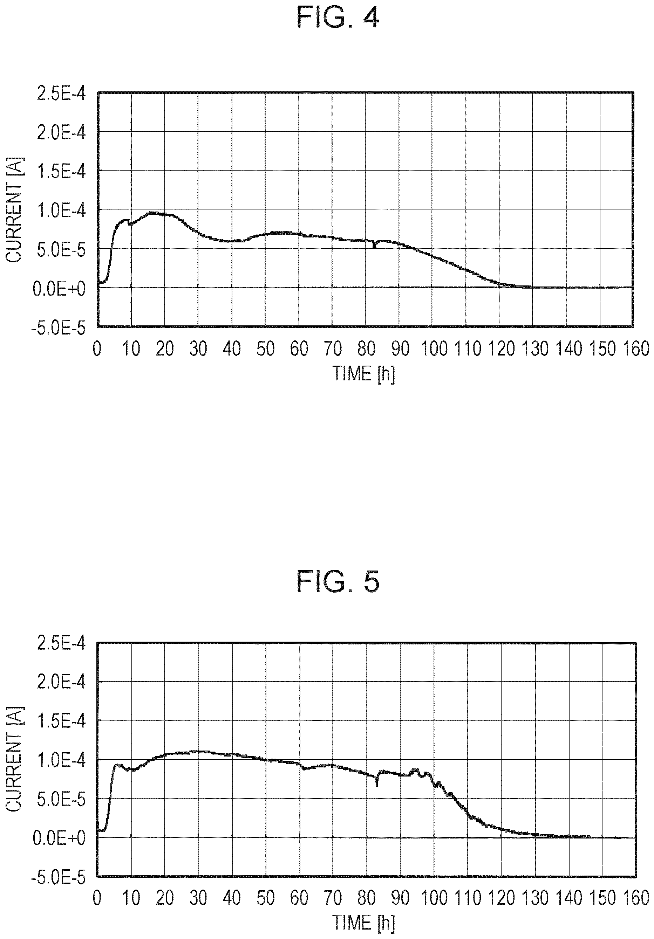

Fig. 4 is a graph showing the relationship between the time during which the voltage was applied to the working electrode and the measured value of the current flowing through the counter electrode in the device of Example 2. In Fig. 4, the vertical axis represents the measured value of the current flowing through the counter electrode, and the horizontal axis represents the time during which the voltage was applied to the working electrode. As shown in Fig. 4, the current flowing through the counter electrode after about 90 hours from the start of the voltage application was gradually decreased, and the current flowing through the counter electrode after about 130 hours from the start of the voltage application was decreased to substantially zero. The concentration of glucose in the sampled treatment liquid was confirmed with a urine test strip every 50 hours from the start of the voltage application. The glucose was clearly detected until 100 hours had elapsed from the start of the voltage application. However, the urine sugar inspection part of the urine test strip did not show a change in color until 150 hours had passed from the start of the voltage application, and this result confirmed that the glucose was decomposed to a concentration less than or equal to the detection limit.

### <Comparative Example 1>

A device of Comparative Example 1 was prepared in the same manner as in Example 2 except that the counter electrode was disposed in the first tank as in an electrochemical reaction device 1b shown in Fig. 2. In addition, 8.1 mL of PBS was accommodated in the second tank of the device of Comparative Example 1 and 8.1 mL of the treatment liquid of Example 2 was accommodated in the first tank of the device of Comparative Example 1 in the same manner as in Example 2.

A constant voltage was continuously applied to the working electrode in the device of Comparative Example 1 in the same manner as in Example 2. In this case, the current flowing through the counter electrode was measured. Fig. 5 is a graph showing the relationship between the time during which the voltage was applied to the working electrode and the measured value of the current flowing through the counter electrode in the device of Comparative Example 1. In Fig. 5, the vertical axis represents the measured value of the current flowing through the counter electrode, and the horizontal axis represents the time during which the voltage was applied to the working electrode. As shown in Fig. 5, the current flowing through the counter electrode after about 100 hours from the start of the voltage application was gradually decreased, and the current flowing through the counter electrode after about 140 hours from the start of the voltage application was decreased to substantially zero. The sampled treatment liquid after 150 hours from the start of the voltage application did not change the color of the urine sugar inspection part of the urine test strip, and this result confirmed that the glucose in the treatment liquid was decomposed to a concentration less than or equal to the detection limit.

It was confirmed that the glucose contained in egg white was decomposed in the device of Example 2 and the device of Comparative Example 1. Further, the current flowing until the glucose was decomposed to a concentration less than or equal to the detection limit in the device of Comparative Example 1 was generally greater than the current flowing until the glucose was decomposed to a concentration less than or equal to the detection limit in the device of Example 2. In Example 2 and Comparative Example 1, the amount of charge required to decompose the glucose was calculated by integrating the current flowing through the counter electrode with time until the glucose in the treatment liquid was decomposed to a concentration less than or equal to the detection limit from the start of the voltage application. The amount of charge in Example 2 was about 25.8 coulombs, whereas the amount of charge in Comparative Example 1 was about 36.5 coulombs. Therefore, the amount of charge required to decompose the glucose in Comparative Example 1 was about 1.4 times the amount of charge required to decompose the glucose in Example 2. A difference between the potential of the working electrode and the potential of the reference electrode was +1.0 V in both the device of Example 2 and the device of Comparative Example 1. Therefore, the energy efficiency of the glucose decomposition in the device of Comparative Example 1 was found to be only about 70% of the energy efficiency of the glucose decomposition in the device of Example 2.

In the device of Example 2, reduced NAD (NADH) can be returned to oxidized NAD (NAD⁺) on the surface of the working electrode by applying the voltage between the working electrode and the counter electrode. Therefore, the glucose decomposition can be continuously carried out. Meanwhile, a potential opposite to that of the working electrode is applied to the counter electrode. Accordingly, in the device of Comparative Example 1, excessive energy is considered to be required due to the occurrence of a reaction in which oxidized NAD (NAD⁺) is changed to reduced NAD (NADH) on the surface of the working electrode.

The treatment liquid after the treatment of Comparative Example 1 was discolored to a slightly blackish color as compared with the treatment liquid after the treatment of Example 2. Since the counter electrode was disposed to be in contact with the treatment liquid in the device of Comparative Example 1, there was a possibility of occurrence of side reactions involving some components in the egg white on the surface of the counter electrode. In the device of Example 2, the inside of the second tank where the counter electrode was disposed and PBS was accommodated and the inside of the first tank where the treatment liquid was accommodated were separated from each other by a membrane. Therefore, it was considered that the components contained in the treatment liquid did not reach the surface of the counter electrode, and thus the side reactions estimated to occur in the device of Comparative Example 1 did not occur. The discoloration of egg white may be a factor of damaging the product value. Therefore, the device of Example 2 was confirmed to have a configuration advantageous for performing an electrochemical decomposition method for glucose contained in egg white.

### <Comparative Example 2>

A device of Comparative Example 2 was prepared in the same manner as in Example 2. A treatment liquid of Comparative Example 2 was prepared in the same manner as in Example 2 except that 0.8 mL of PBS to which no NAD had been added was used in place of the NAD solution. The treatment liquid was accommodated in the first tank and PBS was accommodated in the second tank of the device of Comparative Example 2 in the same manner as in Example 2 except that the treatment liquid of Comparative Example 2 was used in place of the treatment liquid of Example 2.

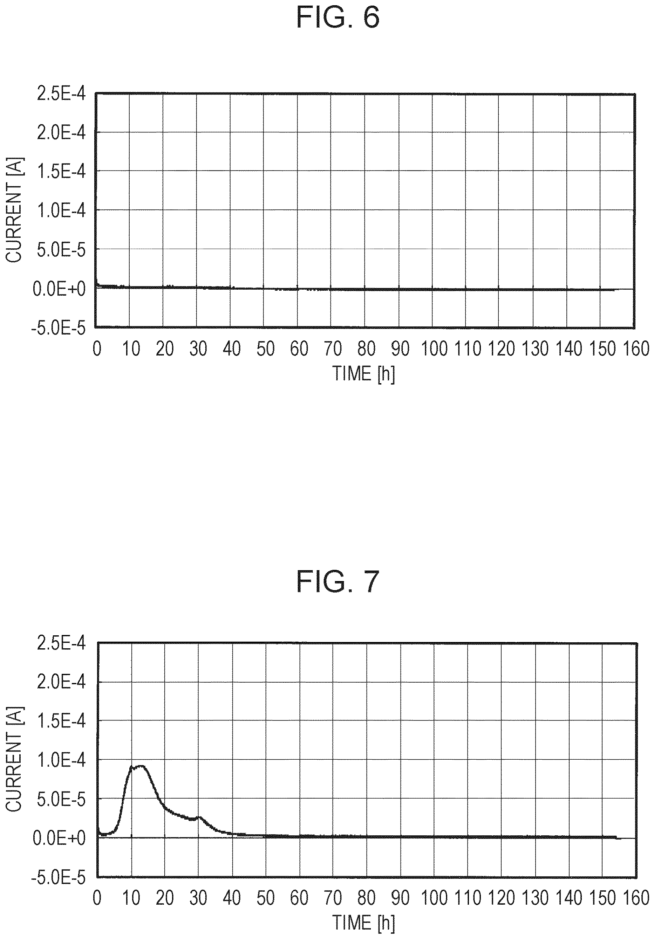

A constant voltage was continuously applied to the working electrode in the device of Comparative Example 2 in the same manner as in Example 2. In this case, the current flowing through the counter electrode was measured. Fig. 6 is a graph showing the relationship between the time during which the voltage was applied to the working electrode and the measured value of the current flowing through the counter electrode in the device of Comparative Example 2. In Fig. 6, the vertical axis represents the measured value of the current flowing through the counter electrode, and the horizontal axis represents the time during which the voltage was applied to the working electrode. As shown in Fig. 6, almost no current flowed through the counter electrode until about 150 hours had passed from the start of the voltage application. It was confirmed that glucose remained in the sampled treatment liquid based on the urine test strip after 150 hours from the start of the voltage application. It was considered that in a case where no NAD had been added to the treatment liquid containing egg white, components donating electrons to electrodes were not present and thus reactions associated with electron transfer did not occur even when the voltage was applied between the working electrode and the counter electrode.

### <Comparative Example 3>

A device of Comparative Example 3 was prepared in the same manner as in Example 2. A treatment liquid of Comparative Example 3 was prepared in the same manner as in Example 2 except that 0.1 mL of PBS to which no GDH had been added was used in place of the GDH solution. The treatment liquid was accommodated in the first tank and PBS was accommodated in the second tank of the device of Comparative Example 3 in the same manner as in Example 2 except that the treatment liquid of Comparative Example 3 was used in place of the treatment liquid of Example 2.

A constant voltage was continuously applied to the working electrode in the device of Comparative Example 3 in the same manner as in Example 2. In this case, the current flowing through the counter electrode was measured. Fig. 7 is a graph showing the relationship between the time during which the voltage was applied to the working electrode and the measured value of the current flowing through the counter electrode in the device of Comparative Example 3. In Fig. 7, the vertical axis represents the measured value of the current flowing through the counter electrode, and the horizontal axis represents the time during which the voltage was applied to the working electrode. As shown in Fig. 7, the current flowed through the counter electrode until about 40 minutes had passed from the start of the voltage application, but since then, almost no current flowed through the counter electrode until about 150 hours had passed from the start of the voltage application. It was confirmed that glucose remained in the sampled treatment liquid based on the urine test strip after 150 hours from the start of the voltage application.

The current generated during the period from the start of the voltage application until about 40 hours had passed is considered to be the current required to convert reduced NAD (NADH) contained in the treatment liquid to oxidized NAD (NAD⁺). Since components donating electrons to electrodes were not present after the entire NAD was oxidized in the treatment liquid, it was considered that the current stopped flowing through the counter electrode after about 40 hours from the start of the voltage application. Therefore, it was considered that almost no oxidoreductases using oxidized NAD (NAD⁺) as a coenzyme were present in egg white and thus products were not also generated from the reactions involving such oxidoreductases.

### Industrial Applicability

The electrochemical reaction device of the present disclosure is advantageous in terms of electrochemical decomposition of glucose.

### Reference Signs List

- 1a: electrochemical reaction device
- 10: first tank
- 11: working electrode
- 16: membrane
- 20: second tank
- 21: counter electrode
- 30: voltage application device

## Claims

1. An electrochemical reaction device comprising:
a first tank in which glucose dehydrogenase and nicotinamide adenine dinucleotide are accommodated and a working electrode is disposed;
a second tank in which a counter electrode is disposed;
a membrane which separates an inside of the first tank and an inside of the second tank from each other, blocks permeation of the glucose dehydrogenase and the nicotinamide adenine dinucleotide, and has ion conductivity; and
a voltage application device which applies a voltage between the working electrode and the counter electrode.

2. The electrochemical reaction device according to claim 1, wherein
the membrane contains a polymer containing a sulfonic acid group in a perfluoro side chain.

3. The electrochemical reaction device according to claim 1 or 2,
wherein the working electrode has a surface formed of platinum.

4. The electrochemical reaction device according to any one of claims 1 to 3, wherein
the counter electrode has a surface formed of platinum.

5. The electrochemical reaction device according to any one of claims 1 to 4,
wherein
the first tank and the second tank accommodate a phosphate buffer solution.

6. An electrochemical decomposition method for glucose, comprising:
applying a voltage between a working electrode disposed in a first tank in which a liquid containing glucose dehydrogenase, nicotinamide adenine dinucleotide, and glucose is accommodated, and a counter electrode,
wherein the counter electrode is disposed in a second tank separated from an inside of the first tank by a membrane that blocks permeation of the glucose dehydrogenase and the nicotinamide adenine dinucleotide and has ion conductivity.
